## Europäisches Patentamt
## European Patent Office
## Office européen des brevets

(19)

(11) Numéro de publication: **0 079 869**
**A2**

# (12) DEMANDE DE BREVET EUROPEEN

(21) Numéro de dépôt: **82830282.8**

(22) Date de dépôt: **16.11.82**

(51) Int. Cl.³: **A 61 F 9/00**

(30) Priorité: **17.11.81 IT 957081**
**30.04.82 IT 938682**

(43) Date de publication de la demande:
**25.05.83 Bulletin 83/21**

(84) Etats contractants désignés:
**AT BE CH DE FR GB LI LU NL SE**

(71) Demandeur: **Cinelli, Arrigo**
**Vialle dei Mille 170**
**I-50133 Firenze(IT)**

(72) Inventeur: **Cinelli, Arrigo**
**Vialle dei Mille 170**
**I-50133 Firenze(IT)**

(74) Mandataire: **Martini, Lazzaro**
**Ufficio Brevetti Ing. Lazzaro Martini Via Brunelleschi, 1**
**I-50123 Firenze(IT)**

(54) Dispositif d'application personnel de verres de contact.

(57) Pour appliquer personnellement ses verres de contact, on utilise un dispositif comprenant: un support d'appui de l'oeil, constitué par deux cylindres (30) horizontaux, à axes parallèles et espacés, tournant verticalement en sens contraire, sur commande d'une poignée (36) afin d'écarter les paupières et découvrir ainsi la cornée; un support du verre de contact devant être appliqué sur la cornée ainsi découverte, constitué par un plateau (20) avec une tige (16), un trou (22) d'alignement et un disque (24) de manoeuvre.

Fig. 3

Fig. 2

## Dispositif d'application personnelle des verres·de contact.

L'invention concerne un dispositif pour appliquer personnellement des verres de contact.

On sait, surtout les personnes portant des verres de contact, que les verres de contact doivent être retirés périodiquement et que les remettre, surtout personnellement, est une opération assez délicate, souvent difficile et requérant à chaque fois un temps plutôt long.

La présente invention a pour but de remédier à ces inconvénients en proposant un dispositif permettant d'appliquer personnellement les verres de contact. Nous avons atteint ce résultat selon l'invention en résoudant deux problèmes:l'un consistant à découvrir la cornée de l'oeil où doit être appliqué le verre et ce, en utilisant deux cylindres d'appui des paupières, espacés, à axes parallèles et tournant verticalement en sens contraire sur commande manuelle;

- 2 -

0079869

l'autre consistant à rapprocher le verre de la cornée ainsi découverte en utilisant un plateau, mobile manuellement, aligné et orienté par rapport à l'oeil.

Les avantages obtenus grâce à cette invention consistent essentiellement en ce que l'application est corecte, rapide et sans aucun problème; en ce que l'application est bonne pour les deux yeux; en ce que n'importe qui peut appliquer tout type de verres de contact; en ce que le dispositif est peu encombrant, d'emploi pratique et simple, de réalisation aisée et de coût réduit.

Dans ce qui suit, l'invention est exposée plus en détail à l'aide des dessins en annexe représentant seulement un mode d'exécution.

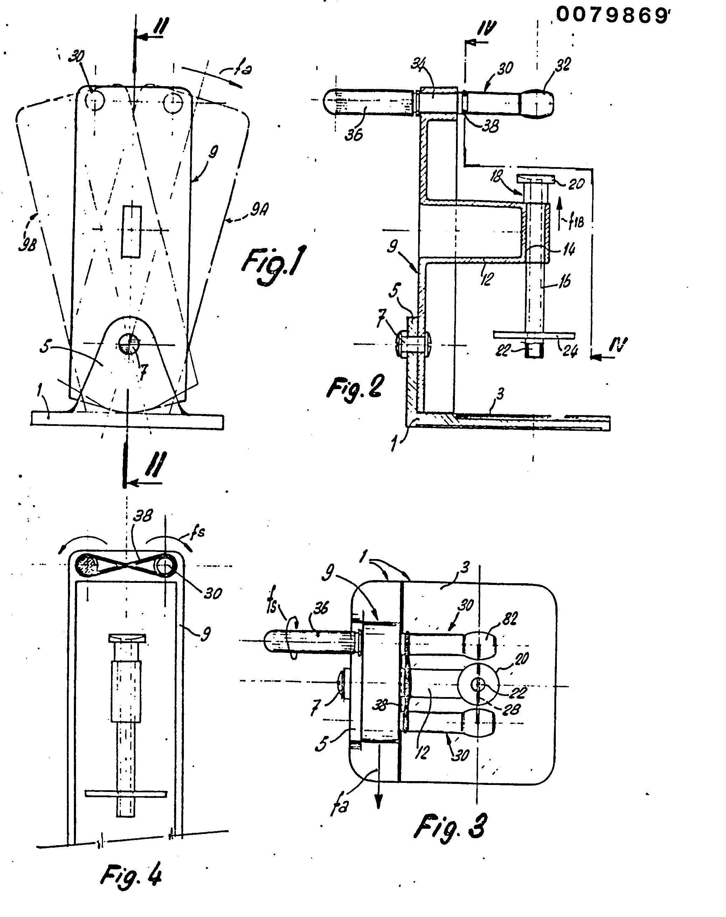

'La figure 1 est la vue latérale d'un dispositif selon une première forme de réalisation; la figure 2 est la vue en coupe selon la ligne II-II de la figure 1; la figure 3 est la vue d'en haut du dispositif de la figure 1, la figure 4 est la vue en coupe selon la ligne IV-IV de la figure 2.

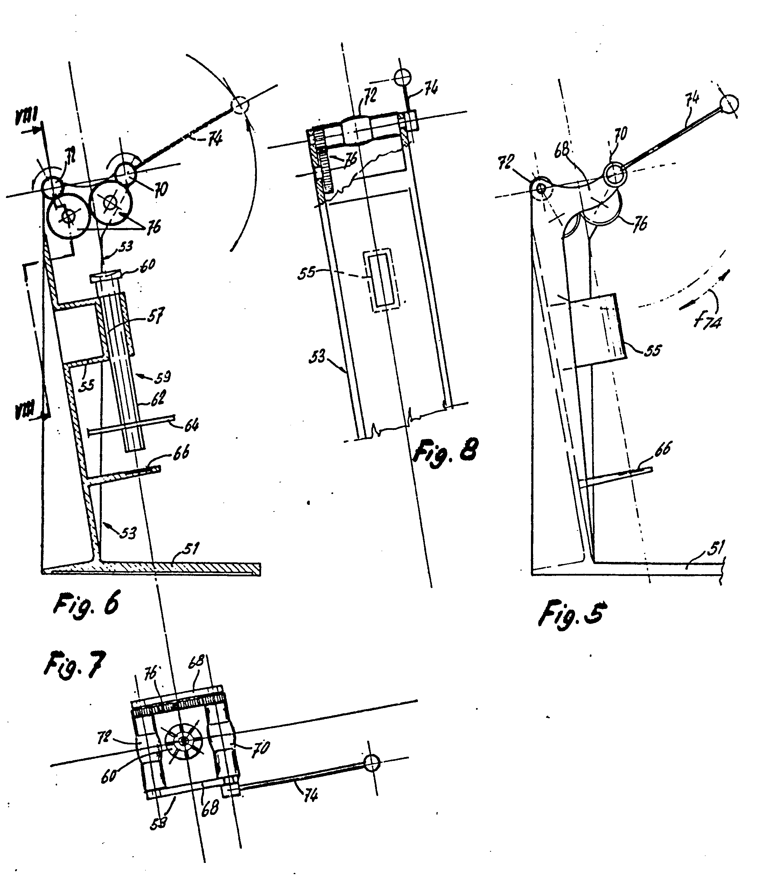

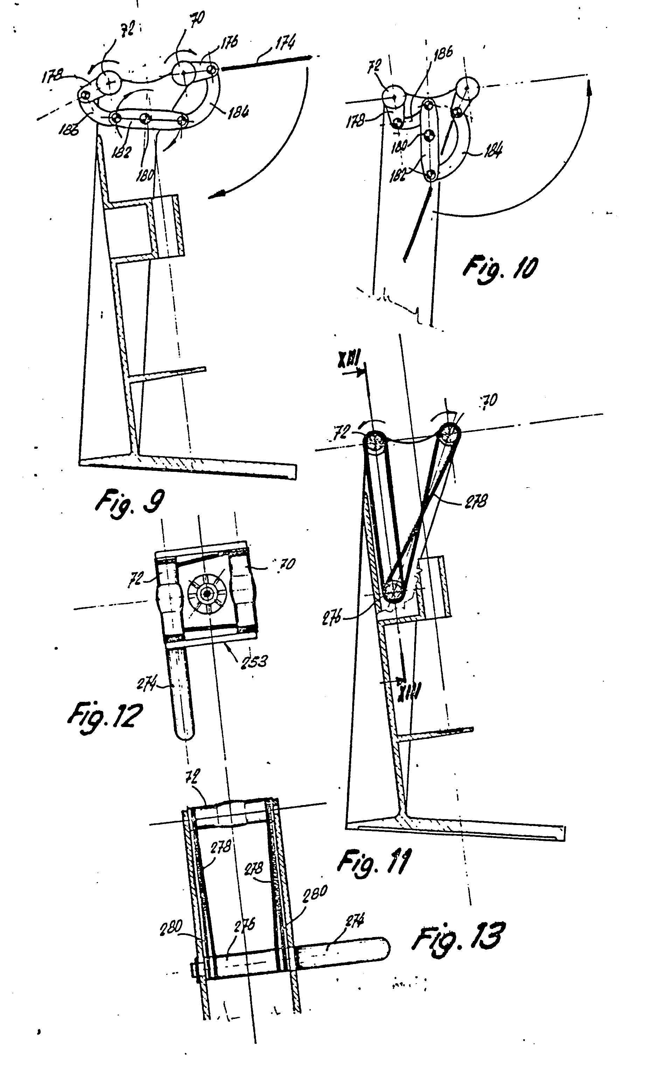

La figure 5 est la vue latérale d'un dispositif selon une deuxième forme de réalisation de l'invention; la figure 6 est la vue en coupe verticale du dispositif de la figure 5; la figure 7 est la vue d'en haut partielle du dispositif de la figure 5; la figure 8 est la vue en coupe selon la ligne VIII-VIII de la figure 6.

La figure 9 est la vue latérale avec une coupe partielle verticale d'une variante du dispositif des figures de 5 à 8; la figure 10 représente le dispositif de la figure 9 en position différente.

- 3 -

0079869

La figure 11 est la vue en coupe verticale d'une autre variante du dispositif des figures de 5 à 8; la figure 12 est la vue d'en haut partielle du dispositif de la figure 11; la figure 13 est la vue en coupe selon la ligne XIII-XIII de la figure 11. Par référence aux figures de 1 à 4 des dessins en annexe, un dispositif d'application personnelle de verres de contact, selon l'invention, est constitué par:

- un socle (1) muni d'un miroir (3) d'où s'élève un support (5) à angle droit.

- un montant (9) s'articulant au moyen d'un pivot (7) sur le support (5) afin de pouvoir osciller entre deux positions extrêmes (9A et 9B) inclinées en sens contraire par rapport à la verticale passant par (7); ce montant est muni d'un appendice ou queue (12) avec un logement (14) passant.

- un groupe (18) porte-verres avec une tige (16) coulissant dans le logement (14) et dont l'extrémité supérieure est munie d'un plateau (20) dont le bord renferme le verre de contact à appliquer: ce plateau (20) peut être remplacé selon les dimensions du verre qu'il supporte. Un trou (22) d'alignement télescopique passe au travers du plateau (20) et de la tige (16): au cours des déplacements du montant (9) entre les deux positions extrêmes (9A-9B), l'axe du trou reste dans un plan perpendiculaire au miroir (3) et au pivot (7) et son repère sur le socle (1) est délimité sur le miroir (3) par une ligne de visée (28). Ce groupe (18) porte-verres s'élève dans le sens de la flèche f18 en coulissant dans le logement (14) par manoeuvre manuelle d'un disque (24) fixé à proximité de l'extrémité inférieure de la tige (16).

- deux cylindres (30) horizontaux, parallèles, espacés et tournant dans les logements relatifs prévus au sommet du montant (9) duquel ils dépassent; leur extrémité libre est en position symétrique par rapport à l'axe de la tige (16) et présente en plus une expansion (32) en forme d'olive se développant symétriquement par rapport au plan de déplacement de l'axe de la tige (16) susmentionnée; un des cylindres (30) se prolonge du montant (9) du côté opposé à l'expansion (32) de façon à former une poignée de manoeuvre (36); le deuxième cylindre (30) est relié au premier par une courroie (38) entrecroisée pour tourner en sens inverse.

Pour appliquer le verre de contact sur l'oeil gauche, après avoir fait tourner le montant (9) dans le sens de la flèche jusqu'à la position inclinée (9A), on approche l'oeil gauche des expansions (32) de façon à voir la ligne de visée (28) du miroir (3) à travers le trou (22) et, tout en maintenant cette vision, on abaisse encore plus la tête de façon à appuyer les paupières sur les expansions (32); puis, à l'aide de la main gauche on tourne la poignée (36) dans le sens de la flèche fs obtenant ainsi, par écartement des paupières, le découvrement de la cornée; en dernier lieu, manoeuvrant le disque (24) de la main droite, on élève le plateau (20) de support du verre se trouvant en dessous des expansions (32) jusqu'à obtenir l'application délicate du verre sur la cornée ainsi découverte. Le verre de contact peut avoir été posé sur le plateau (20) lorsque celui-ci était situé en dessous ou temporairement au-dessus des expansions (32). Pour appliquer ensuite le verre de
contact sur l'oeil droit, après avoir fait tourner
le socle (1) de 180° sur l'horizontale, on porte
le montant (9) en position (9B), on centre l'oeil
droit sur la ligne de visée (28) à travers le trou
(22), on appuie l'oeil sur les expansions (32), de
la main droite on tourne la poignée (36) dans le
sens de la flèche fs et, de la main gauche, on élève le disque (24) jusqu'à ce que le verre de contact, posé sur le plateau (20), adhère à la cornée.
Par référence aux figures de 5 à 8 des dessins en
annexe, un dispositif selon l'invention, est constitué par:

- un socle (51) muni d'un montant (53) incliné par
rapport à la verticale, en position rigide par rapport au socle (51), et muni d'un appendice (55)
avec un logement (57) passant et de deux oreilles
(68) supérieures;

- un groupe (59) porte-verres avec une tige (62)
perforée et coulissant dans le siège (57) susmentionné, dont l'extrémité supérieure est munie d'un plateau (60) de support du verre et l'extrémité inférieure comprend un disque (64) de manoeuvre;

- une patte (66) avec une ligne de visée servant au
centrage de l'oeil sur le verre de contact;

- deux cylindres (70-72) avec une expansion centrale en forme d'olive, montés horizontaux, parallèles, à distance l'un de l'autre et tournant dans
les logements correspondants des oreilles (68) susdites; de plus, ces arbres sont reliés entre-eux par des roues dentées (70) de rapport de transmission égal ou mieux, inférieur à un, pour que l'arbre (72), devant agir sur la paupière inférieure, puisse effectuer une course angulaire plus petite que celle imposée à l'arbre (70) agissant sur la paupière supérieure et ce dans la mesure où, par nature, la paupière supérieure doit effectuer des courses plus grandes que celles de la paupière inférieure. Le cylindre (70) ou celui d'une des roues (76) dépasse en dehors d'une des oreilles (68) du montant (53) de façon à être solidaire d'un levier (74) de manoeuvre avec des déplacements dans le sens de la double flèche f74. Le fonctionnement, similaire à celui du dispositif précédent, est toutefois plus facile dans la mesure ou il n' y a pas besoin de bouger l!appareil en passant d'un oeil à l'autre.

Les figures 9 et 10 des dessins en annexe représentent une variante du dispositif illustré sur les figures de 5 à 8, dans lequel les deux cylindres (70) et (72) sont reliés entre eux par deux manivelles (176-178), deux tiges recourbées (184-186), un levier basculant (182) articulé sur un pivot (180) du montant et dans lequel les cylindres sont manoeuvrés par un levier (174) fixé sur l'arbre (70).

Les figures de 11 à 13 des dessins en annexe représentent une variante ultérieure du dispositif des figures de 5 à 8, dans laquelle le mouvement de rotation des cylindres (70) et (72) dérive d'un troisième arbre (276) situé au-dessous et muni d'une poignée (274) de manoeuvre et par l'intermédiaire d'une ou deux paires de courroies dont une (278) est entrecroisée et l'autre (280) droite.

REVENDICATIONS

1. Dispositif d'application personnelle des verres de contact, caractérisé en ce qu'il comprend: un socle (1) horizontal muni d'un miroir (3) et d'une ligne de visée (28); un montant (9-53) avec deux cylindres (30-70/72) horizontaux, parallèles, placés à distance les uns des autres, servant de support de l'oeil, tournant verticalement en sens contraire, de course radiale différente, et avec un logement (14-57) perforé dont l'axe est biais et symétrique par rapport aux dits cylindres; un groupe porte-verres (18) avec une tige (16-62) mobile dans le logement susmentionné et un plateau supérieur (20-60) pour tenir le verre, tous deux pourvus d'un trou (22) axial servant à l'alignement de l'oeil, du verre de contact et de la ligne de visée (28), et avec un disque (24-64) de manoeuvre.

2. Dispositif selon la revendication 1, caractérisé en ce que le dit montant (9) est articulé avec le socle (1) au moyen d'un pivot (7) horizontal.

3. Dispositif selon la revendication 1, caractérisé en ce que le dit montant (53) est incliné par rapport à la verticale et en position rigide avec le socle (1).

4. Dispositif selon la revendication 1, caractérisé en ce que les dits cylindres (30) sont montés en porte-à-faux, sur un seul côté du montant (9).

5. Dispositif selon la revendication 1, caractérisé en ce que les dits cylindres (70/72) sont montés entre les deux oreilles (68) du dit montant (53).

6. Dispositif selon la revendication 1, caractérisé en ce que les dits cylindres (30-70/72) présentent une expansion (32) en forme d'olive.

7. Dispositif selon la revendication 1, caractérisé en ce que les dits cylindres (30) sont reliés entre eux par une transmission à courroie (38) entrecroisée et que leur rotation est commandée par une poignée (36) situé sur un des dits cylindres (30).

8. Dispositif selon la revendication 1, caractérisé en ce que les dits cylindres (70/72) sont reliés entre eux par une transmission à roues dentées (76) et que leur rotation est commandé par un levier (7) solidaire d'un des cylindres (70/72).

9. Dispositif selon la revendication 1, caractérisé en ce que les dits cylindres (70/72) sont reliés entre eux par une articulation comprenant deux manivelles (176-178), un levier basculant (182) et en ce que leur rotation est commandée par un levier (174) solidaire du cylindre (70).

10. Dispositif selon la revendication 1, caractérisé en ce que les dits cylindres (70/72) sont reliés à un troisième cylindre (276) au moyen d'une transmission comprenant au moins deux courroies dont l'une (278) est entrecroisée et l'autre (280) droite et en ce que leur rotation est commandée par une poignée (274) du dit cylindre (276).

0079869

Fig. 1

Fig. 2

Fig. 4

Fig. 3

Fig. 6

Fig. 8

Fig. 5

Fig. 7

Fig. 9

Fig. 10

Fig. 11

Fig. 12

Fig. 13